# EUROPEAN PATENT APPLICATION

(11) **EP 3 167 844 A1**
(43) Date of publication of application: **17.05.2017**
(21) Application number: 16198062.8
(22) Date of filing: 10.11.2016
(51) Int. Cl.: A61F 2/02

(54) **MALE SPHINCTER PROSTHESIS FOR EXTERNAL USE ON URETHRAL CHANNEL**

(30) Priority: 10.11.2015 ES 201500823
(71) Applicant: Zafra Méndez, José, 18008 Granada (ES)
(72) Inventor: Zafra Méndez, José, 18008 Granada (ES)
(74) Representative: Bartrina Diaz, José María

(57) **Abstract**

External placement prosthesis that acts as a sphincter on the male urinary canal, composed of a single body with a preferably cylindrical-circular structure (1) made entirely of silicone material of different density from that used in the health area. The element exerting the pressure function on the urethra, pyramidal in the manner of a protuberance (4), is located in the lower part of the interior of the device and its task is to impinge on just the urinary duct or urethra, made of density silicone very low, exerting a continuous and even pressure without being excessive. It is envisaged that the inner region of the device at its upper part (7) is covered or also filled with highly silicone material to prevent the device from being able to turn.

## Description

### SECTOR RELATED TO THE ART

As stated in the title of this specification, the proposed invention falls within the field of the sector dedicated to applications, prostheses or devices used by males who through age, having undergone surgery radical prostate surgery, a benign prostate hyperplasia or being seriously impaired, suffer uncontrolled urinary incontinence. The group of people affected is not excluded in any way because of infectious disease, such as cystitis amongst others, and may be assisted in this situation in avoiding the loss of urine through the use of the device presented.

### OBJECT OF THE INVENTION

The present specification is contemplated within the invention patent registration form. It proposes an external prosthesis that acts as the sphincter of the male urinary tract. It contains a protruding feature tasked with pressing upon the urethra or urinary tract, thereby preventing leakage. Built in a very soft, supple and soft to the touch material, such as silicone, it will facilitate the expulsion of urine by only separating lightly with the fingers the top and bottom surrounding the penis in order to remove the pressure that the device exerts on the urethra. It immediately returns to its normal shape and pressure by simply releasing it.

### BACKGROUND OF THE INVENTION

Currently within the field of urology, there are different control devices for male incontinence with differing degrees of success. Such devices may be applicable both internally, which requires surgical intervention for installation, and externally, which is less aggressive, consists of different materials and is mainly inspired by the clamp-crush technique on the urinary tract of the urethra in order to voluntarily obstruct it in order to avoid the output of urine, as is discussed below in greater detail. At a registry level, inventiveness in this field is not excessive, and therefore there does not appear to be a glut of patents or utility models for the purpose outlined; on the other hand, in view of the documents found, they differ considerably from the appliance developed and described in this present specification.

Amongst the inventions found in the different registration databases, especially those based on using the technique of clamping the urinary tract, there are to our judgement different and considerable disadvantages compared to the new invention proposed in this specification, amongst which the following can be set forth:
- U 9002378 **Utility Model Device for controlling male incontinence,** which displays a device composed of two sections, not small in size and constructed of a rigid or semi-rigid material. Considerations about its effectiveness in clamping the urethra successfully aside, the actual structure of this device renders its use very uncomfortable and in no way secure, and it may cause some kind of injury around the area where it is placed.
- European Patent No. 05746796.1 **PENILE COMPRESSION DEVICE,** where a form of pressure on the entire penis and urethra area is proposed through a diffuse technique to which another feature is added as a kind of sleeve in a rigid material, and can even be metallic, with the associated risk that this may involve for use in the area of the body in question.
- **Patent No.** 200802479 **Transformable external controller device for urinary incontinence.** This consists of an external device belonging to the pharmaceutical sector for controlling male urinary incontinence. It has the form of a flattened ring shape which, when pressed laterally with two fingers, is transformed into a circular ring allowing urination without removing it from the penis or dismantling it. **Its flexibility is enabled by *two articulated steel springs.***
- **International Patent** D/806843 **"Cunningham clip,"** describes a clamp for a "clip" feature made of metallic material, where the clip exerts pressure upon the penile area which over time may not be free of pain.

The proposed invention is an advancement and improvement upon the state of the art, overcoming the drawbacks of known devices, especially in the form and strength of the pressure exerted in order to achieve urinary tract obstruction. One of its innovations of the device that is the subject of the present patent is that pressure only directly affects the urinary tract, leaving the rest of the area free of tightness. What it in fact does is to wrap or subject it with the purpose of the device being held securely in its original position and unable to be rotated, change position or effectively be moved. It consists of a single body by adopting a circular-cylindrical structure, made entirely of a different density silicone material, as appropriate for each feature of the device structure. The outer edge of the structure is circular, built in higher density silicone and therefore more rigid but simultaneously elastic, unlike the main body that is used to build a lower density and softer silicone. In the inner bottom section of the device, there is a small, emerging protuberance-like feature built in very soft silicone material, which presses exactly against the urinary tract or urethra, and owing to properties of the silicon used in its manufacture acts as a buffer, exerting a continuous and uniform pressure without this being excessive, the user hardly being aware of it. It does not damage cavernous tissue as it does not press against it, is easy to put in place and is used when evacuating urine (its usage will be explained presently). By being made entirely of silicone material used in prosthetics and surgical material, i.e.; biocompatible, it allows the patent proposed (unlike others known that are made of solid materials, such as plastics or metal) to be odourless, tasteless, and to not absorb odours, liquids or colour. It is easily washed, does not develop bacteria, is not detectable in arcs or safety systems against metallic features, thus safeguarding the privacy of anyone using it. Any kind of sporting activity may be engaged in with it, such as swimming, running, cycling, etc., and for optimum results its manufacture can be customized.

### DESCRIPTION OF FIGURES

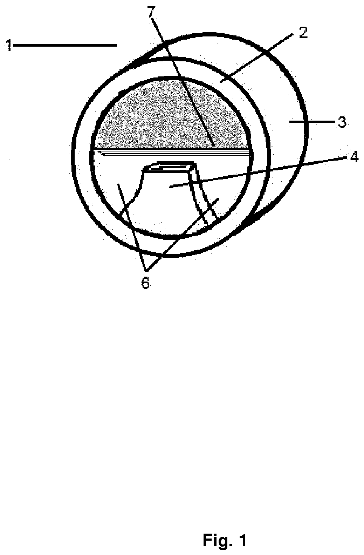

Figure 1 shows a front elevation isometric view of all the features that make up the device.

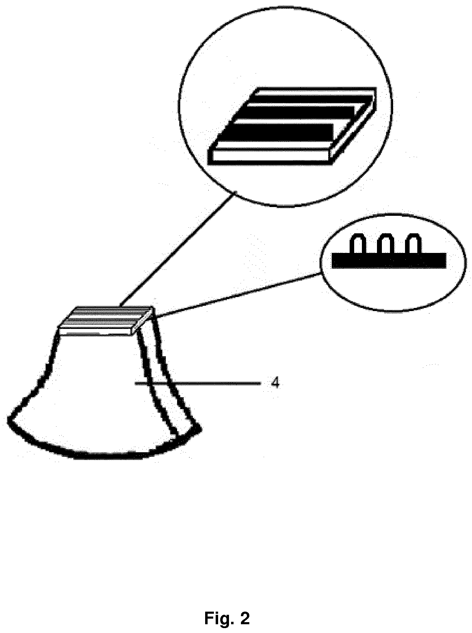

Figure 2 shows a front elevation isometric view of the feature exerting pressure on the function of the urethra, pyramidal and with enlarged detail of the front and side apex where three features of little height protruding over its surface may be observed.

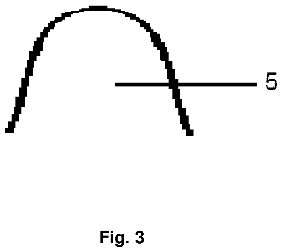

Figure 3 shows a front elevation isometric view with the features that join the device with the feature that serves the pressure function on the urethra in a frustoconical shape and upper surface with a rounded finish.

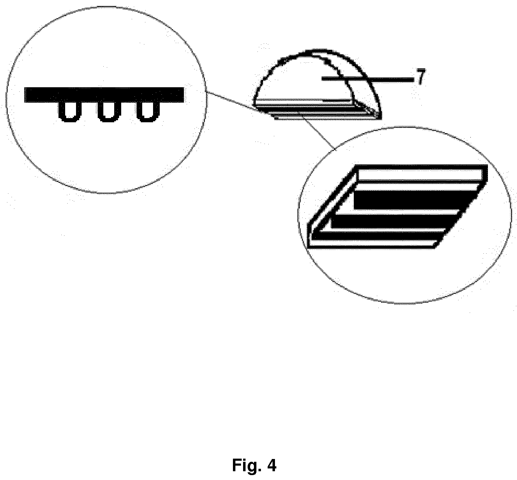

Figure 4 shows a front view of the feature covering or filling the inside of the device, and enlarged detail from its bottom or base on different frontal and lateral perspectives where the three low-height features which protrude over the surface base can be observed.

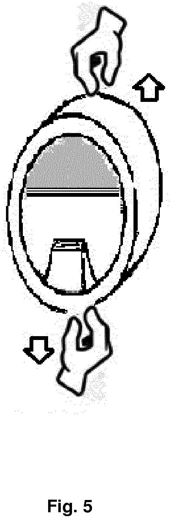

Figure 5 shows a front elevational view of the deformed device when stretched at its top and bottom by using fingers of both hands.

### DESCRIPTION OF PREFERRED PRODUCTION METHOD

The patent proposed by the present specification, as a continuation of the above and in keeping with its title, is an external prosthesis which acts as the sphincter of the male urinary tract in order to improve the quality of life of people who for various reasons, such as illness or age, lead them to suffer urinary incontinence. By using this prosthesis, the output of urine can be perfectly controlled, without being adversely affected by certain discomfort, pain or other external disturbances. The invention consists of a single preferably circular-cylindrical structure (1) measuring approximately 17 mm in length and 42 mm in outside diameter, made entirely of silicone material of different density according to the requirements for each feature making up the device, from its use in the health field, biologically tested for medical products, irritation and skin sensitization tests. The outer contour or edge of the structure adopts a circular-cylindrical circular shape, (2) made of a higher density silicone in order to be more consistent, and simultaneously elastic, measuring approximately 6 mm thick. The device body (3) is also manufactured in lower density silicon and as a result has a softer texture of fine thickness, allowing a perfect fit over the penis without rendering such pressure excessive. The feature that performs the function of pressure on the urethra is pyramidal shaped as a protuberance (4) and is located at the lower inside part the device; its purpose is to affect just the urinary tract or urethra. It is therefore one of the invention's essential features, is made of silicone like other features employed in medical products. The feature's very low density allows it to act like a cushion or pad exerting a continuous and uniform pressure without this being excessive, so that the user hardly perceives it. According to the user's own morphology, this feature can take two different forms: a quasi-pyramidal structure (4), on which three features that are low in height project along its width, and on its top or apex, also built in a very soft silicone material, spaced equidistantly in order to cover the total area, and the three exerting pressure on the urethra. The purpose of these three features is designed to raise the security setting, in order that if it should be maladjusted and not apply pressure to the urethra for whatever reason, other features will perform this function, increasing security as has been stated, and optimizing the objective of preventing involuntary urine output. The other way will adopt a more frustoconical shape, with the top part acting upon the smooth and the rounded, smooth and wider urethra (5). The use of the device will not harm the cavernous tissue of the penis, because that area is free of pressure (6). In order to avoid the device being moved or rotated once the user has positioned it, with the result that the press-on features (4 or 5) have no efficient impact on the urethra, it is anticipated that the inner area of the device in the top part (7) will be covered or also filled by very soft silicone material and occupy a space in the inside area of approximately 13 mm in height, and of a long internal width within the device of approximately 42 mm. Its base or bottom is composed of three low height features made of silicone material, which protrude at equidistant spaces in order to cover the total area. Their function is to seek extra support for the prosthesis by reducing the possibility of it rotating when in use.

In terms of the pressure feature, on the basis of its shape (4 or 5) it will be positioned at a short distance from touching the inner filling (7). Having explained the structure of the device and the material used for each of its parts, the instructions will now be detailed, where simplicity in placing the device is paramount and for the user who, once having used it and with the need to urinate, must simply lift or pull both the lower and upper ring (2) at the edge of the device so that the pressure feature or protuberance (4) no longer presses on the urethra and the top of the inner filling (7) is separated from the penis, allowing urine to flow normally, with the device becoming effective again when simply released.

It is not considered necessary to extend this specification to any person skilled in the art to understand the scope of the invention and the advantages derived from its use. Sizes, shapes, mechanisms and constituent materials of the invention may be varied to suit the advantages that can be derived from its specific application, provided that this does not affect the essence of the invention. The terms in which this present specification should always be taken in an illustrative and non-limiting manner.

## Claims

1. **MALE SPHINCTER PROSTHESIS FOR EXTERNAL USE ON URETHRAL CHANNEL, characterized by** a cylindrical-circular structure (1), constructed entirely of silicone material of the kind used in the health field, biologically tested for medical products, irritation and skin sensitization tests. The outer edge or contour of the cylindrical-circular structure adopts a circular shape, (2) also made of the same silicone material, but with a range with which it acquires properties of a higher consistency, less soft and simultaneously elastic. The body of the device (3) is also made of silicone, but of a softer texture and of a fine thickness, allowing its perfect adhesion on the penis, without exerting pressure on it.

2. **MALE SPHINCTER PROSTHESIS FOR EXTERNAL USE ON URETHRAL CHANNEL, characterized in that** according to claim 1, in the lower inner part there is a pyramidal-structured protrusion (4) made of soft-density silicone. On the surface of its upper part and along its width, three low height features stand out, also constructed of very soft silicon material, and separated from each other equidistantly to cover the total area.

3. **MALE SPHINCTER PROSTHESIS FOR EXTERNAL USE ON URETHRAL CHANNEL, characterized in that** according to claim 1, in its inner lower part there is a frustoconical structure (5) made of soft density silicone, and with the surface of its top part having a round and smooth finish.

4. **MALE SPHINCTER PROSTHESIS FOR EXTERNAL USE ON URETHRAL CHANNEL, characterized in that** according to claim 1, the inner area of the upper part of the device is covered or filled with soft silicone (7) occupying a space within the inner area of approximately 13 mm in height, and approximately 17 mm along the internal width of the device, leaving its base line at a few millimetres from the pressing feature or protuberance (4 or 5).
Its base or bottom is composed of three low features made of silicone material, which protrude separately from each other equidistantly until covering the total area in width.

5. **MALE SPHINCTER PROSTHESIS FOR EXTERNAL USE ON URETHRAL CHANNEL, characterized in that** according to claim 1, the diameter is enlarged by extending the upper part of the ring (2) and lower with the fingers of both hands.
